# EUROPEAN PATENT APPLICATION

(11) **EP 1 731 163 A2**
(43) Date of publication of application: **13.12.2006**
(21) Application number: 06112312.1
(22) Date of filing: 13.10.2003
(51) Int. Cl.: A61K 38/20, A61P 37/00, A61P 33/00

(54) **Treatment of allergic conditions by use of IL 21**

(30) Priority: 11.10.2002 DK 200201546; 16.10.2002 DK 200201587
(62) Divisional of application: 03757715.2
(71) Applicant: NOVO NORDISK A/S, 2880 Bagsvaerd (DK)
(72) Inventor: Roemer, John, 2880, Bagsværd (DK); Moeller, Niels, Peter, Hundahl, 2880, Bagsværd (DK); Skak, Kresten, 2880, Bagsværd (DK)
(74) Representative: Winther, Kamilla

(57) **Abstract**

The invention relates to management, treatment and prevention of allergic reactions, allergic diseases and allergic conditions or parasitic diseases by administration of IL-21.

## Description

### FIELD OF THE INVENTION

The invention relates to management, treatment and prevention of allergic reactions, allergic diseases and allergic conditions by administation of IL-21. Further, the invention relates to treatment of parasitic diseases by administering IL-21 to patients infected with parasites.

### BACKGROUND OF THE INVENTION

IL-21, which has also been termed Zalpha11, is a cytokine, which was shown to be produced by activated CD4+ T lymphocytes after stimulation with anti-CD3, phorbol ester plus ionomycin (Parrish-Novak et al., *Nature 408*, 57 - 63 (2000)).

Numerous studies have shown that eosinophils play a central in the development of asthma and other allergic diseases (reviewed in Foster et al., *TRENDS in Molecular Medicine 8,* 162 - 167 (2002)). Eosinophils are produced from stem cells in the bone marrow ('eosinopoiesis') in response to IL-5 and eotaxin, and the eosinophils migrate via the blood to the lungs where they are activated. Such activated eosinophils release proinflammatory molecules and granular proteins, which can damage the lung tissue and induce airways hyperresponsiveness. It is widely held that so-called T helper 2 ('T_{H}2') lymphocytes in the lungs orchestrate most cellular reactions via their release of 'T_{H}2 cytokines' (i.e. IL-4, IL-5, IL-9, IL-10 and IL-13). According to this view, eosinophils and basophils act as effector cells only (reviewed in Wills-Karp et al. *Annu. Rev. Immunol. 17,* 255 (1999)). Surprisingly, we have now discovered that IL-21 is produced by, stored in and released by human eosinophilic granulocytes (hereinafter 'eosinophils'). IL-21 has been shown to act as a co-stimulatory cytokine for proliferation of T and B lymphocytes (Parrish-Novak et al., *supra).* Thus, eosinophils may in addition to their role as primary effector cells, which damage the respiratory epithelium, also play another ― to this point unrecognized ― protective effect by releasing IL-21. Released IL-21 from eosinophils (hereinafter 'eosinophilic IL-21') may in turn either directly, or in concert with other cytokines, prevent differentiation and activation of cells involved in allergic reactions and thereby contribute to a less severe allergic reaction. According to this view, eosinophils may play a dual role in allergic reactions. On one hand the release granular proteins from eosinophils can damage tissues, but on the other hand release of eosinophilic IL-21 may reduce the allergic reactions via an effect on other cells. Asthma has been increasing in prevalence, morbidity, and mortality over the last two decades, and there is a strong need for improved treatment of asthma and other allergic diseases.

The above brief description of the central role of eosinophils in asthma is believed to apply to allergic reactions in general. A list of allergic conditions or diseases, which is not intended in anyway to limit the scope of the invention, include asthma, anaphylaxis, drug reactions, food allergy, insect venom allergy, allergic rhinitis, urticaria, eczema, atopic dermatitis, allergic contact allergy, allergic conjunctivitis. Such allergic reaction, allergic diseases or allergic conditions may be managed, treated or prevented by administering IL-21.

Eosinophils are further important for combating parasitic diseases including but not limited to helminthic infections.

The present invention relates to a method for treating, preventing and/or managing allergic diseases or conditions by administration of IL-21.

### SUMMARY OF THE INVENTION

It has been found that eosinophils synthesize, store and/or secrete IL-21. Eosinophils may be involved in the protective response to allergic reactions, diseases and conditions. The invention relates to managing, treating and preventing allergic reactions, conditions and diseases by administration of IL-21.

### DEFINITIONS

A "polypeptide" is a polymer of amino acid residues linked by peptide bonds, and may be produced naturally or synthetically. Polypeptides of less than about 10 amino acid residues are commonly referred to as "peptides".

A "protein" is a macromolecule comprising one or more polypeptide chains, which may be produced naturally or synthetically. A protein may also comprise non-peptidic components, such as carbohydrate groups or other non-peptidic substituents. Carbohydrates and other non-peptidic substituents may be added to a protein by the cell in which the protein is produced, and will vary with the type of cell. Carbohydrates and other non-peptidic substituents may also be added synthetically after the cell-based production of the protein. Proteins are defined herein in terms of their amino acid backbone structures; substituents such as carbohydrate groups or other non-peptidic substituents are generally not specified, but may be present nonetheless.

International Patent Application No. PCT/US06067, publication no. WO 00/53761, published September 14, 2000, which is hereby incorporated in this application in its entirety, discloses IL-21 (as "cytokine zalpha11 ligand") as SEQ ID No. 2, which is hereby incorporated in this application in its entirety, and which is also shown as SEQ ID No. 2 in this application, as well as methods for producing it and antibodies thereto and a polynucleotide sequence encoding IL-21 as SEQ ID No. 1. The present invention also contemplates the use of IL-21 polypeptides which as used herein should be taken to mean polypeptides with a sequence identity to the polypeptide of SEQ ID No: 2, or their orthologs comprising at least 70%, at least 80%, at least 90%, at least 95%, or greater than 95%. The present invention also includes the use of polypeptides that comprise an amino acid sequence having at least 70%, at least 80%, at least 90%, at least 95% or greater than 95% sequence identity to the sequence of amino acid residues 1 to 162, residues 30 to 162, or residues 33 to 162 of SEQ ID No: 2. Methods for determining percent identity are described below. The IL-21 polypeptides of the present invention have retained all or some of the biological activity of IL-21 which makes IL-21 useful for treating allergic reactions, allergic diseases and allergic conditions. Some of the polypeptides may also have a biological activity which is higher than the biological activity of IL-21.

"Eosinophilic cells", "eosinophilic granulocytes" or "eosinophils" are defined as leukocytes that contain red-staining eosinophil granules (e.g. with Wright's or Hematoxylin & Eosin stain). Eosinophils are involved both in normal physiological reactions and in disease processes. Included in the definition, but not limited to this definition, are (i) eosinophils in the bone marrow, (ii) eosinophils circulating in peripheral blood, (iii) eosinophils involved allergic reactions (including but not limited to asthma, anaphylaxis, drug reactions, food allergy, insect venom allergy, allergic rhinitis, urticaria, eczema, atopic dermatitis, allergic contact allergy, allergic conjunctivitis), (iv) eosinophils involved in malignant diseases (with Hodgkin's lymphoma, mycosis fungoides, chronic myelogenous leukemia, and cancer of the lung, stomach, pancreas, ovary or uterus as non-limiting examples), (v) eosinophils involved in autoimmune reactions, (vi) eosinophils involved in collagen vascular diseases (with rheumatoid arthritis and periarteritis as non-limiting examples), (vii) eosinophils involved in helminthic infections, and/or (vii) eosinophils involved in iatrogenic disorders. Also included are eosinophils in Loeffler's syndrome, eosinophilia-myalgia syndrome and idiopathic hypereosinophilic syndromes.

"Eosinophilic IL-21 ", as used herein, should be taken to mean IL-21 that is produced by, stored in and/or secreted by eosinophilic granulocytes.

The term "treatment" and "treating" as used herein means the management and care of a patient for the purpose of combating a condition, such as a disease or a disorder. The term is intended to include the full spectrum of treatments for a given condition from which the patient is suffering, such as prevention of the condition, the delaying of the progression of the disease, disorder or condition, the alleviation or relief of symptoms and complications, and/or the cure or elimination of the disease, disorder or condition. The patient to be treated is preferably a mammal, in particular a human being.

### DESCRIPTION OF THE DRAWINGS

### Figure 1a

*In situ* hybridization showing expression of IL-21 mRNA in a specimen of Hodgkin's Lymphoma with eosinophilic infiltration. Images of the same field are shown as brightfield in the left panels and as darkfield in the right panels. The signal for IL-21 mRNA is seen as bright spots in the darkfield images.

### Figure 1b

*In situ* hybridization showing expression of IL-21 mRNA in eosinophils in a specimen of Hodgkin's Lymphoma with eosinophilic infiltration. In the high magnification picture at right the signal for IL-21 mRNA is visible as black silver grains over eosinophils (arrows).

### Figure 2

*In situ* hybridization showing expression of IL-21 mRNA in a specimen of Hodgkin's Lymphoma with lymphocytic predominance. Images of the same field are shown as brightfield in the left panel and as darkfield in the right panel. The signal for IL-21 mRNA is seen over lymphocytes as bright spots surrounding the Reed-Sternberg cell (arrow) in the darkfield image.

### Figure 3

*In situ* hybridization showing weak expression of IL-21 receptor mRNA in specimens of Hodgkin's Lymphoma with eosinophilic infiltration (upper panels) or with lymphocytic predominance (lower panels). Images of the same field are shown as brightfield in the left panels and as darkfield in the right panels. The weak signal for IL-21 R mRNA is seen diffusely all over the tissue as bright spots in the darkfield images.

### Figure 4

SEQ ID NO. 1: DNA sequence encoding IL-21.

### Figure 5

SEQ ID NO. 2: amino acid sequence of IL-21.

### DESCRIPTION OF THE INVENTION

It has been found that eosinophils synthesize, store and/or secrete IL-21. Eosinophils may be involved in the protective response to allergic reactions, diseases and conditions.

In one embodiment, the invention relates to treating diseases or conditions where eosinophils are involved in a protective response, including but not limited to allergic reactions or conditions, by administration of IL-21 to a subject in need thereof.

In one embodiment, the invention relates to treating allergic reactions, conditions and diseases by administration of IL-21 to a subject in need thereof.

In one embodiment, the invention relates to treating diseases or conditions where eosinophils are involved in a protective response, including but not limited to allergic reactions or conditions, by administration of IL-21 polypeptides that have a sequence identity to the polypeptide of SEQ ID No: 2, or their orthologs of at least 70%, at least 80%, at least 90%, at least 95%, or greater than 95%. The present invention also includes the use of polypeptides that comprise an amino acid sequence having at least 70%, at least 80%, at least 90%, at least 95% or greater than 95% sequence identity to the sequence of amino acid residues 1 to 162, residues 30 to 162, or residues 33 to 162 of SEQ ID No: 2. Methods for determining percent identity are described below. The polypeptides of the present invention having a sequence identity to the polypeptide of SEQ ID No: 2, or their orthologs as described above have retained all or some of the biological activity of IL-21 which makes IL-21 useful for treating allergic reactions, allergic diseases and allergic conditions.

In one embodiment, the invention relates to treating allergic reactions, conditions and diseases by administration of IL-21 polypeptides that have a sequence identity to the polypeptide of SEQ ID No: 2, or their orthologs of at least 70%, at least 80%, at least 90%, at least 95%, or greater than 95%. The present invention also includes the use of polypeptides that comprise an amino acid sequence having at least 70%, at least 80%, at least 90%, at least 95% or greater than 95% sequence identity to the sequence of amino acid residues 1 to 162, residues 30 to 162, or residues 33 to 162 of SEQ ID No: 2. Methods for determining percent identity are described below. The polypeptides of the present invention having a sequence identity to the polypeptide of SEQ ID No: 2, or their orthologs as described above have retained all or some of the biological activity of IL-21 which makes IL-21 useful for treating allergic reactions, allergic diseases and allergic conditions.

In one embodiment, the invention relates to treating diseases or conditions where eosinophils are involved, including but not limited to allergic reactions or conditions, by administration of an IL-21 mimetic, that is a compound which is not an IL-21 polypeptide as described above, but which has the biological activity of IL-21. An IL-21 mimetic may be a peptide, such as a polypeptide or an oligopeptide or may be non-proteins, such as a smaller organic molecule.

In one embodiment, the invention relates to treating diseases or conditions where eosinophils are involved in a protective response, including but not limited to allergic reactions or conditions, by administration of an IL-21 mimetic, that is a compound which is not an IL-21 polypeptide as described above, but which has the biological activity of IL-21. An IL-21 mimetic may be a peptide, such as a polypeptide or an oligopeptide or may be non-proteins, such as a smaller organic molecule.

In one embodiment, the invention relates to treating diseases or conditions where eosinophils are involved in a protective response, including but not limited to allergic reactions or conditions, by administration of a polynucleotide encoding IL-21 or a IL-21 polypeptide that have a sequence identity to the polypeptide of SEQ ID No: 2, or their orthologs, of at least 70%, at least 80%, at least 90%, at least 95%, or greater than 95%. In a further embodiment, the present invention relates to managing, treating and preventing allergic reactions, conditions and diseases by administration of a polynucleotide encoding polypeptides that comprise an amino acid sequence having at least 70%, at least 80%, at least 90%, at least 95% or greater than 95% sequence identity to the sequence of amino acid residues 1 to 162, residues 30 to 162, or residues 33 to 162 of SEQ ID No: 2. An example of such a polynucleotide is shown as SEQ ID No. 1 coding for a polypeptide with a sequence as shown in SEQ ID No. 2.

In one embodiment, the invention relates to treating allergic reactions, conditions and diseases by administration of a polynucleotide encoding IL-21 or a IL-21 polypeptide that have a sequence identity to the polypeptide of SEQ ID No: 2, or their orthologs, of at least 70%, at least 80%, at least 90%, at least 95%, or greater than 95%. In a further embodiment, the present invention relates to managing, treating and preventing allergic reactions, conditions and diseases by administration of a polynucleotide encoding polypeptides that comprise an amino acid sequence having at least 70%, at least 80%, at least 90%, at least 95% or greater than 95% sequence identity to the sequence of amino acid residues 1 to 162, residues 30 to 162, or residues 33 to 162 of SEQ ID No: 2.

Percentage sequence identity between two amino acid sequences is determined by a Needelman-Wunsch alignment, useful for both protein and DNA alignments. For protein alignments the default scoring matrix used is BLOSUM50, and the penalty for the first residue in a gap is -12, while the penalty for additional residues in a gap is -2. The alignment may be made with the Align software from the FASTA package version v20u6 (W.R. Pearson and D.J. Lipman (1988), "Improved Tools for Biological Sequence Analysis", PNAS 85:2444-2448; and W.R. Pearson (1990) "Rapid and Sensitive Sequence Comparison with FASTP and FASTA", Methods in Enzymology, 183:63-98).

IL-21 or other IL-21 polypeptides for use in treating asthma or other allergic conditions according to the present invention may be administered alone or in combination with other established therapies such as β₂-agonists, cromolyn, leukotriene receptor antagonists, corticosteroidcytokines, cytokine antagonists, interleukins and interleukin antagonists, which are only provided as non-limiting examples.

### PHARMACEUTICAL COMPOSITIONS

IL-21 or other IL-21 polypeptides for use in treating asthma or other allergic conditions according to the present invention may be administered alone or in combination with pharmaceutically acceptable carriers or excipients, in either single or multiple doses. The pharmaceutical compositions comprising IL-21 or other IL-21 polypeptides for use in treating asthma or other allergic conditions according to the present invention may be formulated with pharmaceutically acceptable carriers or diluents as well as any other known adjuvants and excipients in accordance with conventional techniques such as those disclosed in Remington: The Science and Practice of Pharmacy, 19^{th} Edition, Gennaro, Ed., Mack Publishing Co., Easton, PA, 1995. The compositions may appear in conventional forms, for example capsules, tablets, aerosols, solutions or suspensions

The pharmaceutical compositions may be specifically formulated for administration by any suitable route such as the oral, rectal, nasal, pulmonary, topical (including buccal and sublingual), transdermal, intracisternal, intraperitoneal, vaginal and parenteral (including subcutaneous, intramuscular, intrathecal, intravenous and intradermal) route. It will be appreciated that the preferred route will depend on the general condition and age of the subject to be treated, the nature of the condition to be treated and the active ingredient chosen. The route of administration may be any route, which effectively transports the active compound to the appropriate or desired site of action.

Pharmaceutical compositions for oral administration include solid dosage forms such as hard or soft capsules, tablets, troches, dragees, pills, lozenges, powders and granules. Where appropriate, they can be prepared with coatings such as enteric coatings or they can be formulated so as to provide controlled release of the active ingredient such as sustained or prolonged release according to methods well known in the art.

Liquid dosage forms for oral administration include solutions, emulsions, aqueous or oily suspensions, syrups and elixirs.

Pharmaceutical compositions for parenteral administration include sterile aqueous and non-aqueous injectable solutions, dispersions, suspensions or emulsions as well as sterile powders to be reconstituted in sterile injectable solutions or dispersions prior to use. Depot injectable formulations are also contemplated as being within the scope of the present invention.

Other suitable administration forms include suppositories, sprays, ointments, cremes, gels, inhalants, dermal patches, implants etc.

A typical oral dosage is in the range of from about 0.001 to about 100 mg/kg body weight per day, such as from about 0.01 to about 50 mg/kg body weight per day, for example from about 0.05 to about 10 mg/kg body weight per day administered in one or more dosages such as 1 to 3 dosages. The exact dosage will depend upon the nature of the IL-21 polypeptide chosen, the frequency and mode of administration, the sex, age, weight and general condition of the subject treated, the nature and severity of the condition treated and any concomitant diseases to be treated and other factors evident to those skilled in the art.

The formulations may conveniently be presented in unit dosage form by methods known to those skilled in the art. A typical unit dosage form for oral administration one or more times per day such as 1 to 3 times per day may contain from 0.05 to about 1000 mg, for example from about 0.1 to about 500 mg, such as from about 0.5 mg to about 200 mg.

For parenteral routes such as intravenous, intrathecal, intramuscular and similar administration, typically doses are in the order of about half the dose employed for oral administration.

Non-protein IL-21 mimetics for use in treating asthma or other allergic conditions according to the present invention are generally utilized as the free substance or as a pharmaceutically acceptable salt thereof. Examples are an acid addition salt of a compound having the utility of a free base and a base addition salt of a compound having the utility of a free acid. The term "pharmaceutically acceptable salts" refers to non-toxic salts of such compounds which are generally prepared by reacting the free base with a suitable organic or inorganic acid or by reacting the acid with a suitable organic or inorganic base. When such a compound contains a free base such salts are prepared in a conventional manner by treating a solution or suspension of the compound with a chemical equivalent of a pharmaceutically acceptable acid. When such a compound contains a free acid such salts are prepared in a conventional manner by treating a solution or suspension of the compound with a chemical equivalent of a pharmaceutically acceptable base. Physiologically acceptable salts of a compound with a hydroxy group include the anion of said compound in combination with a suitable cation such as sodium or ammonium ion. Other salts which are not pharmaceutically acceptable may be useful in the preparation of compounds of the invention and these form a further aspect of the invention.

Salts of IL-21 polypeptides are especially relevant when the protein is in solid or crystalline form

For parenteral administration, solutions of the IL-21 polypeptides or IL-21 mimetics in sterile aqueous solution, aqueous propylene glycol or sesame or peanut oil may be employed. Such aqueous solutions should be suitably buffered if necessary and the liquid diluent first rendered isotonic with sufficient saline or glucose. The aqueous solutions are particularly suitable for intravenous, intramuscular, subcutaneous and intraperitoneal administration. The sterile aqueous media employed are all readily available by standard techniques known to those skilled in the art.

Suitable pharmaceutical carriers include inert solid diluents or fillers, sterile aqueous solution and various organic solvents. Examples of solid carriers are lactose, terra alba, sucrose, cyclodextrin, talc, gelatine, agar, pectin, acacia, magnesium stearate, stearic acid and lower alkyl ethers of cellulose. Examples of liquid carriers are syrup, peanut oil, olive oil, phospholipids, fatty acids, fatty acid amines, polyoxyethylene and water. Similarly, the carrier or diluent may include any sustained release material known in the art, such as glyceryl monostearate or glyceryl distearate, alone or mixed with a wax. The pharmaceutical compositions formed by combining a IL-21 polypeptide or IL-21 mimetic for use in treating asthma or other allergic conditions according to the present invention and the pharmaceutically acceptable carriers are then readily administered in a variety of dosage forms suitable for the disclosed routes of administration. The formulations may conveniently be presented in unit dosage form by methods known in the art of pharmacy.

For nasal administration, the preparation may contain a IL-21 polypeptide or IL-21 mimetic dissolved or suspended in a liquid carrier, in particular an aqueous carrier, for aerosol application. The carrier may contain additives such as solubilizing agents, e.g. propylene glycol, surfactants, absorption enhancers such as lecithin (phosphatidylcholine) or cyclodextrin, or preservatives such as parabenes.

Formulations of IL-21 polypeptides or IL-21 mimetics for use in treating asthma or other allergic conditions according to the present invention suitable for oral administration may be presented as discrete units such as capsules or tablets, each containing a predetermined amount of the active ingredient, and which may include a suitable excipient. Furthermore, the orally available formulations may be in the form of a powder or granules, a solution or suspension in an aqueous or non-aqueous liquid, or an oil-in-water or water-in-oil liquid emulsion.

Compositions intended for oral use may be prepared according to any known method, and such compositions may contain one or more agents selected from the group consisting of sweetening agents, flavouring agents, colouring agents, and preserving agents in order to provide pharmaceutically elegant and palatable preparations. Tablets may contain the active ingredient in admixture with non-toxic pharmaceutically-acceptable excipients which are suitable for the manufacture of tablets. These excipients may be for example, inert diluents, such as calcium carbonate, sodium carbonate, lactose, calcium phosphate or sodium phosphate; granulating and disintegrating agents, for example corn starch or alginic acid; binding agents, for example, starch, gelatine or acacia; and lubricating agents, for example magnesium stearate, stearic acid or talc. The tablets may be uncoated or they may be coated by known techniques to delay disintegration and absorption in the gastrointestinal tract and thereby provide a sustained action over a longer period. For example, a time delay material such as glyceryl monostearate or glyceryl distearate may be employed. They may also be coated by the techniques described in U.S. Patent Nos. 4,356,108; 4,166,452; and 4,265,874, incorporated herein by reference, to form osmotic therapeutic tablets for controlled release.

Formulations for oral use may also be presented as hard gelatine capsules where the active ingredient is mixed with an inert solid diluent, for example, calcium carbonate, calcium phosphate or kaolin, or a soft gelatine capsules wherein the active ingredient is mixed with water or an oil medium, for example peanut oil, liquid paraffin, or olive oil.

Aqueous suspensions may contain the IL-21 polypeptides or IL-21 mimetics in admixture with excipients suitable for the manufacture of aqueous suspensions. Such excipients are suspending agents, for example sodium carboxymethylcellulose, methylcellulose, hydroxypropylmethylcellulose, sodium alginate, polyvinylpyrrolidone, gum tragacanth and gum acacia; dispersing or wetting agents may be a naturally-occurring phosphatide such as lecithin, or condensation products of an alkylene oxide with fatty acids, for example polyoxyethylene stearate, or condensation products of ethylene oxide with long chain aliphatic alcohols, for example, heptadecaethyl-eneoxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyethylene sorbitan monooleate. The aqueous suspensions may also contain one or more colouring agents, one or more flavouring agents, and one or more sweetening agents, such as sucrose or saccharin.

Oily suspensions may be formulated by suspending the active ingredient in a vegetable oil, for example arachis oil, olive oil, sesame oil or coconut oil, or in a mineral oil such as a liquid paraffin. The oily suspensions may contain a thickening agent, for example beeswax, hard paraffin or cetyl alcohol. Sweetening agents such as those set forth above, and flavouring agents may be added to provide a palatable oral preparation. These compositions may be preserved by the addition of an anti-oxidant such as ascorbic acid.

Dispersible powders and granules suitable for preparation of an aqueous suspension by the addition of water provide the active compound in admixture with a dispersing or wetting agent, suspending agent and one or more preservatives. Suitable dispersing or wetting agents and suspending agents are exemplified by those already mentioned above. Additional excipients, for example, sweetening, flavouring, and colouring agents may also be present.

The pharmaceutical compositions of IL-21 polypeptides or IL-21 mimetics for use in treating asthma or other allergic conditions according to the present invention may also be in the form of oil-in-water emulsions. The oily phase may be a vegetable oil, for example, olive oil or arachis oil, or a mineral oil, for example a liquid paraffin, or a mixture thereof. Suitable emulsifying agents may be naturally-occurring gums, for example gum acacia or gum tragacanth, naturally-occurring phosphatides, for example soy bean, lecithin, and esters or partial esters derived from fatty acids and hexitol anhydrides, for example sorbitan monooleate, and condensation products of said partial esters with ethylene oxide, for example polyoxyethylene sorbitan monooleate. The emulsions may also contain sweetening and flavouring agents.

Syrups and elixirs may be formulated with sweetening agents, for example glycerol, propylene glycol, sorbitol or sucrose. Such formulations may also contain a demulcent, preservatives and flavouring and colouring agents. The pharmaceutical compositions may be in the form of a sterile injectible aqueous or oleaginous suspension. This suspension may be formulated according to the known methods using suitable dispersing or wetting agents and suspending agents described above. The sterile injectable preparation may also be a sterile injectable solution or suspension in a non-toxic parenterally-acceptable diluent or solvent, for example as a solution in 1,3-butanediol. Among the acceptable vehicles and solvents that may be employed are water, Ringer's solution, and isotonic sodium chloride solution. In addition, sterile, fixed oils are conveniently employed as solvent or suspending medium. For this purpose, any bland fixed oil may be employed using synthetic mono- or diglycerides. In addition, fatty acids such as oleic acid find use in the preparation of injectables.

The compositions may also be in the form of suppositories for rectal administration of the compounds of the invention. These compositions can be prepared by mixing the drug with a suitable non-irritating excipient which is solid at ordinary temperatures but liquid at the rectal temperature and will thus melt in the rectum to release the drug. Such materials include cocoa butter and polyethylene glycols, for example.

For topical use, creams, ointments, jellies, solutions of suspensions, etc., containing the compounds of the invention are contemplated. For the purpose of this application, topical applications shall include mouth washes and gargles.

The IL-21 polypeptides or IL-21 mimetics for use in treating asthma or other allergic conditions according to the present invention may also be administered in the form of liposome delivery systems, such as small unilamellar vesicles, large unilamellar vesicles, and multilamellar vesicles. Liposomes may be formed from a variety of phospholipids, such as cholesterol, stearylamine, or phosphatidylcholines.

In addition, some of the IL-21 polypeptides or IL-21 mimetics for use in treating asthma or other allergic conditions according to the present invention may form solvates with water or common organic solvents. Such solvates are also encompassed within the scope of the invention.

If a solid carrier is used for oral administration, the preparation may be tabletted, placed in a hard gelatine capsule in powder or pellet form or it can be in the form of a troche or lozenge. The amount of solid carrier will vary widely but will usually be from about 25 mg to about 1 g. If a liquid carrier is used, the preparation may be in the form of a syrup, emulsion, soft gelatine capsule or sterile injectable liquid such as an aqueous or non-aqueous liquid suspension or solution.

The IL-21 polypeptides or IL-21 mimetics for use in treating asthma or other allergic conditions according to the present invention may be administered to a mammal, especially a human, in need of such treatment. Such mammals include also animals, both domestic animals, e.g. household pets, and non-domestic animals such as wildlife.

Pharmaceutical compositions containing a compound according to the invention may be administered one or more times per day or week, conveniently administered at mealtimes. An effective amount of such a pharmaceutical composition is the amount that provides a clinically significant effect. Such amounts will depend, in part, on the particular condition to be treated, age, weight, and general health of the patient, and other factors evident to those skilled in the art.

### EXAMPLES

### Expression of IL-21 and IL-21 R mRNA in Hodgkin Lymphoma

*In situ* hybridization analysis of tissue micro array slides (DAKO tumor multislide, DAKO cat. No. T1064) has revealed that IL-21 and IL-21 R mRNA are expressed in Hodgkin Lymphoma specimens. The signal is seen in several specimens of Hodgkins disease derived from different patients. All other types of cancers that are represented on the multislide (approximately 20 different types) show no expression of IL-21 or IL-21 R mRNA.

### Probe preparation, hybridization conditions and Stringency washing:

### Probe preparation:

In situ hybridization is performed with a probe generated by in vitro transcription from a plasmid containing the following cDNA fragment: A PCR fragment of human IL-21 containing basepair 344 to basepair 833 (acc.no.: AF254069 (se Table A)) containing a single mismatch compared to the published sequence was cloned into the transcription vector pCRBlunt II (Invitrogen).

### Hybridization:

The specific activity of the S-35 labelled probe was 80000 cpm/ml in the final hybridization mixture (consisting of 10 x SALTS, deionised Formamid, 50% dextransulphate, t-RNA (10 mg/ml), 1.0 M DTT). The sections were hybridized overnight at 47°C.

### Stringency wash:

The sections were washed in 50% formamide, 1 x SALTS (300 mM NaCl, 10 mM Tris (pH 6.8), 10 mM NaPO₄, 5 mM EDTA, 0.02% Ficoll 400, 0.02% polyvinylpyrolidone, and 0.02% BSA) and 10 mM dithiothreitiol for 1 hour at 57°C and 1 hour at 62°C. After RNAse A treatment (20 µg/ml RNase A) in 0.5 M NaCl, 10 mM Tris-Cl (pH 7.2), 1 mM EDTA) at 37°C for 30 min., the sections were washed for 30 min. in 0.1 x SSC at room temperature. The sections were dipped in autoradiographic emulsion and exposed for 21 days.

### Results:

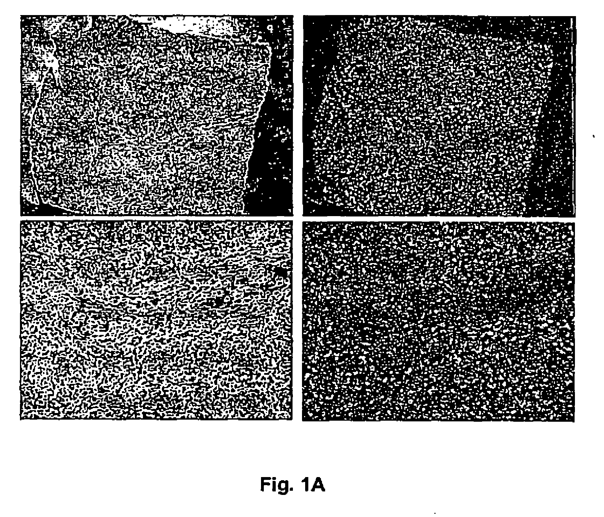

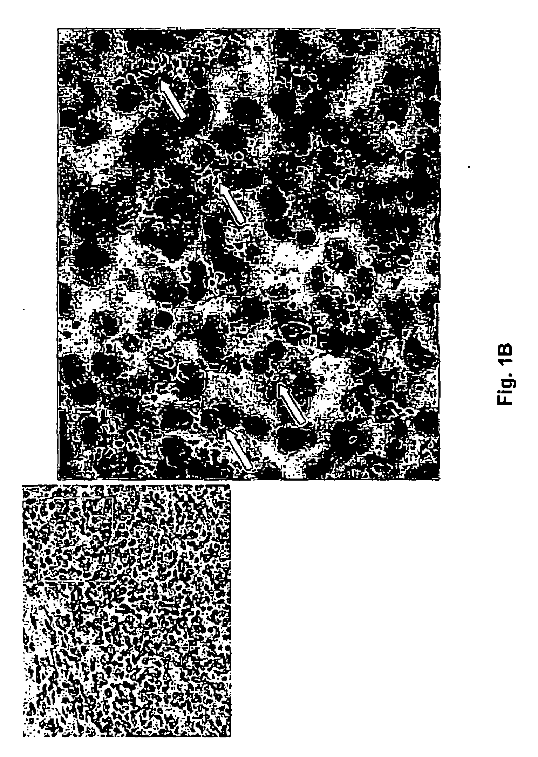

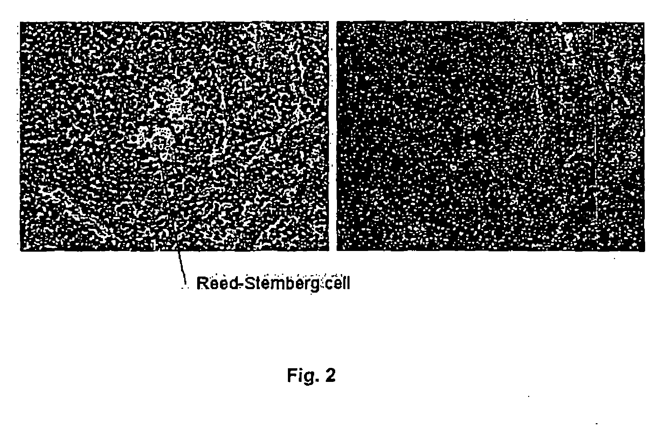

In the specimens belonging to the histomorphological defined subgroup of Hodgkins lymphoma with eosinophilic infiltration, IL-21 mRNA is expressed predominantly in eosinophilic granulocytes (Figure 1 a). Following in situ hybridization the sections are counterstained with hematoxylin and eosin (H&E) whereby it is possible to morphologically identify the IL-21 mRNA positive cells as eosinophils (arrows in figure 1 b). In specimens with lymphocytic predominance expression of IL-21 mRNA is found in small clusters of lymphocytes surrounding Reed-Sternberg cells (figure 2)

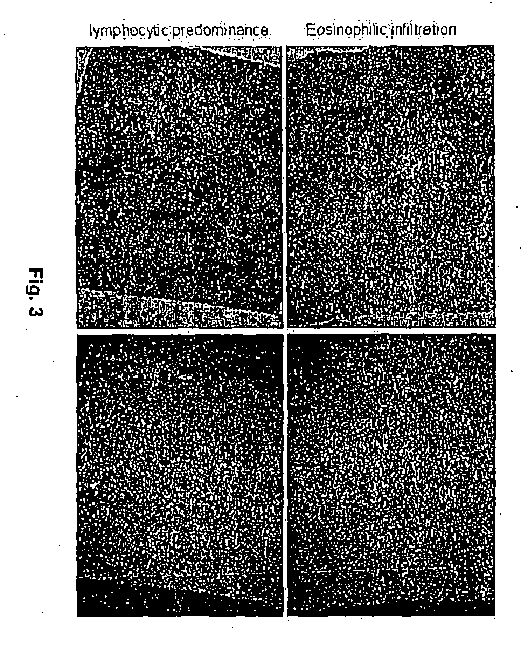

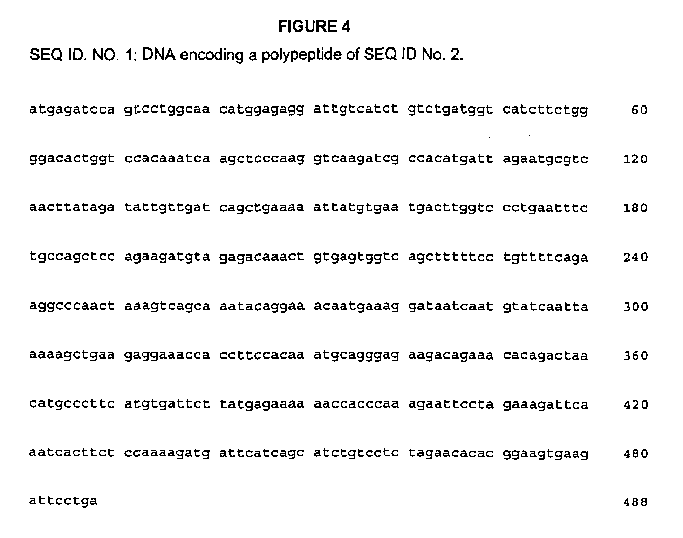

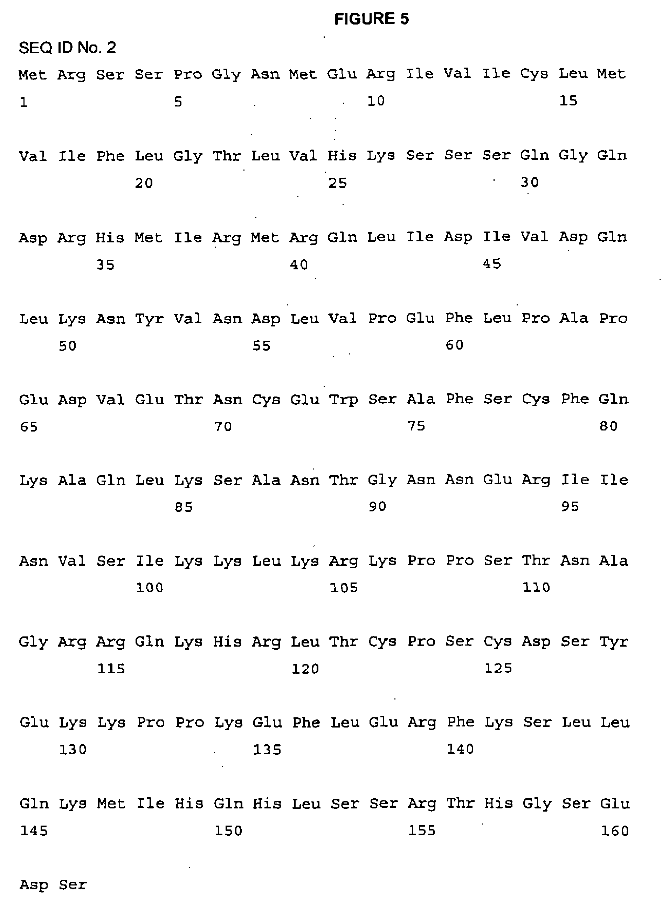

### IL-21 R mRNA is diffusely expressed in lymphocytes in both the eosinophilic and lymphocytic types of histopathology (figure 3)

### Conclusion:

The expression of IL-21 in eosinophils may indicate that said eosinophilic IL-21 exerts a protective role. According to this view, eosinophilic infiltration or increased number of eosinophils in body fluids and/or tissues in certain disorders or disease states or conditions may indicate that said eosinophils play a protective role by releasing IL-21, which in turn reduces an allergic reaction or allergic disease. Thus, diseases where eosinophils are involved in a protective role, including but not limited to allergic diseases may be treated by administration of IL-21 polypeptides or IL-21 mimetics.

IL-21 and the IL-21 receptor may play a role in the patho-physiology of Hodgkin's Lymphoma, and IL-21 signalling may therefore be a target for pharmacological intervention in Hodgkin's disease.

Particularly the distinct expression of IL-21 in lymphocytes immediately surrounding the malignant Reed-Sternberg cells indicate that the IL-21 mRNA expression is involved in a cross-talk between Reed-Sternberg cells and surrounding lymphocytes and the IL-21 expression may even be induced by the Reed-Sternberg cells.

A method to demonstrate IL-21 and IL-21 R expression in an eosinophil-like cell-line, HL-60: HI-60 cells are cultured in the presence of butyric acid to induce an eosinophil-like phenotype, and are subsequently cultured with cytokines or growth factors, non limiting examples are GM-CSF, eotaxin, IL-4, IL-5 and IFN-γ, and harvested after 1-72 hrs culture and analysed for IL-21 and IL-21 R mRNA expression by RT-PCR and for IL-21 protein expression by ELISA and IL-21 and IL-21 R protein expression by flow cytometry

A method to demonstrate IL-21 and IL-21 R expression in eosinophils: Eosinophils are isolated from human blood and cultured with cytokines or growth factors as described in Woerly et al., *J.Leukoc.Biol.* 72:769-779, 2002; Schmid-Grendelmeier et al., *J Immunol* 169:1021-1027, 2002, non limiting examples are GM-CSF, eotaxin, IL-4, IL-5 and IFN-γ, and harvested after 1-72 hrs culture and analysed for IL-21 and IL-21 R mRNA expression by RT-PCR and for IL-21 protein expression by ELISA and IL-21 and IL-21 R protein expression by flow cytometry.

A method to demonstrate abnormal expression of IL-21 and IL-21 R in eosinophils from patients with allergic diseases: Eosinophils are isolated from the blood from patients with allergic diseases and from healthy donors and analysed directly ex vivo for IL-21 and IL-21 R expression by RT-PCR, ELISA or flow cytometry, or stimulated with growth factors or cytokines before analysis of IL-21 and IL-21 R expression as described above.

A method to demonstrate abnormal expression of IL-21 and IL-21 R in eosinophils from patients with allergic diseases: Biopsies from patients with allergic diseases and from healthy donors challenged with relevant antigens are sectioned are stained by immunohistochemistry for IL-21 and IL-21 R expression together with a relevant marker to define the cell-type, for example EG-2 as an eosinophil marker.

A method to demonstrate that absence of IL-21 leads to increased disease severity in a mouse model of asthma: IL-21 deficient and wild type mice are immunized with ovalbumin and subsequently challenged by intranasal instillation of ovalbumin, and the airway responsiveness, the cellular composition of the bronchoalveolar lavage, and cytokine, chemokine and IgE levels are measured as described in Hogan et al., *J Immunol* 171:2644-2651 (2003) or Medoff et al., *J Immunol* 168:5278-5286, 2002 or Denzler et al., *J Immunol* 165:5509-5517 (2000) or Ishimitsu et al., *J Immunol* 166:1991-2001 (2001). . An increased disease severity in IL-21 deficient mice indicates that IL-21 may be useful to treat asthma.

A method to demonstrate that neutralization of IL-21 leads to increased disease severity in a mouse model of asthma: Mice are immunized with ovalbumin and subsequently challenged by intranasal instillation of ovalbumin. Before, after or together with ovalbumin challenge, the mice are treated with neutralizing IL-21 antibody. The airway responsiveness, the cellular composition of the bronchoalveolar lavage, and cytokine, chemokine and IgE levels are measured as described in Hogan et al. *J Immunol* 171:2644-2651 (2003) or Medoff et al., *J Immunol* 168:5278-5286 (2002) or Denzler et al., *J Immunol* 165:5509-5517 (2000) or Ishimitsu et al., *J Immunol* 166:1991-2001 (2001). An increased disease severity in mice treated with neutralizing IL-21 antibody indicates that IL-21 may be useful to treat asthma.

A method to demonstrate that IL-21 treatment leads to diminished disease severity in a mouse model of asthma: Mice are immunized with ovalbumin and subsequently challenged by intranasal instillation of ovalbumin. Before, after or together with ovalbumin challenge, the mice are treated with IL-21 protein, a plasmid encoding IL-21 or buffer or control plasmid. The airway responsiveness, the cellular composition of the bronchoalveolar lavage, and cytokine, chemokine and IgE levels are measured as described in Hogan et al., *J Immunol* 171:2644-2651 (2003) or Medoff et al., *J Immunol* 168:5278-5286 (2002) or Denzler et al., *J Immunol* 165:5509-5517 (2000) or Ishimitsu et al., *J Immunol* 166:1991-2001 (2001).

A method to demonstrate that absence of IL-21 leads to increased disease severity in helminthic infections: IL-21 deficient and wild type mice are immunized with killed (frozen) microfilariae followed by i.v. injection with live microfilariae, and the allergic response is measured as described above and in Hall et al., *Infect.Immun*. 66:4425-4430 (1998)

A method to demonstrate that IL-21 may be used to treat helminthic infections: mice are immunized with killed (frozen) microfilariae followed by i.v. injection with live microfilariae. Before, after or together with injection of live microfilariae, the mice are treated with IL-21 protein, a plasmid encoding IL-21 or buffer or control plasmid. The allergic response is measured as described above and in Hall et al. as above.

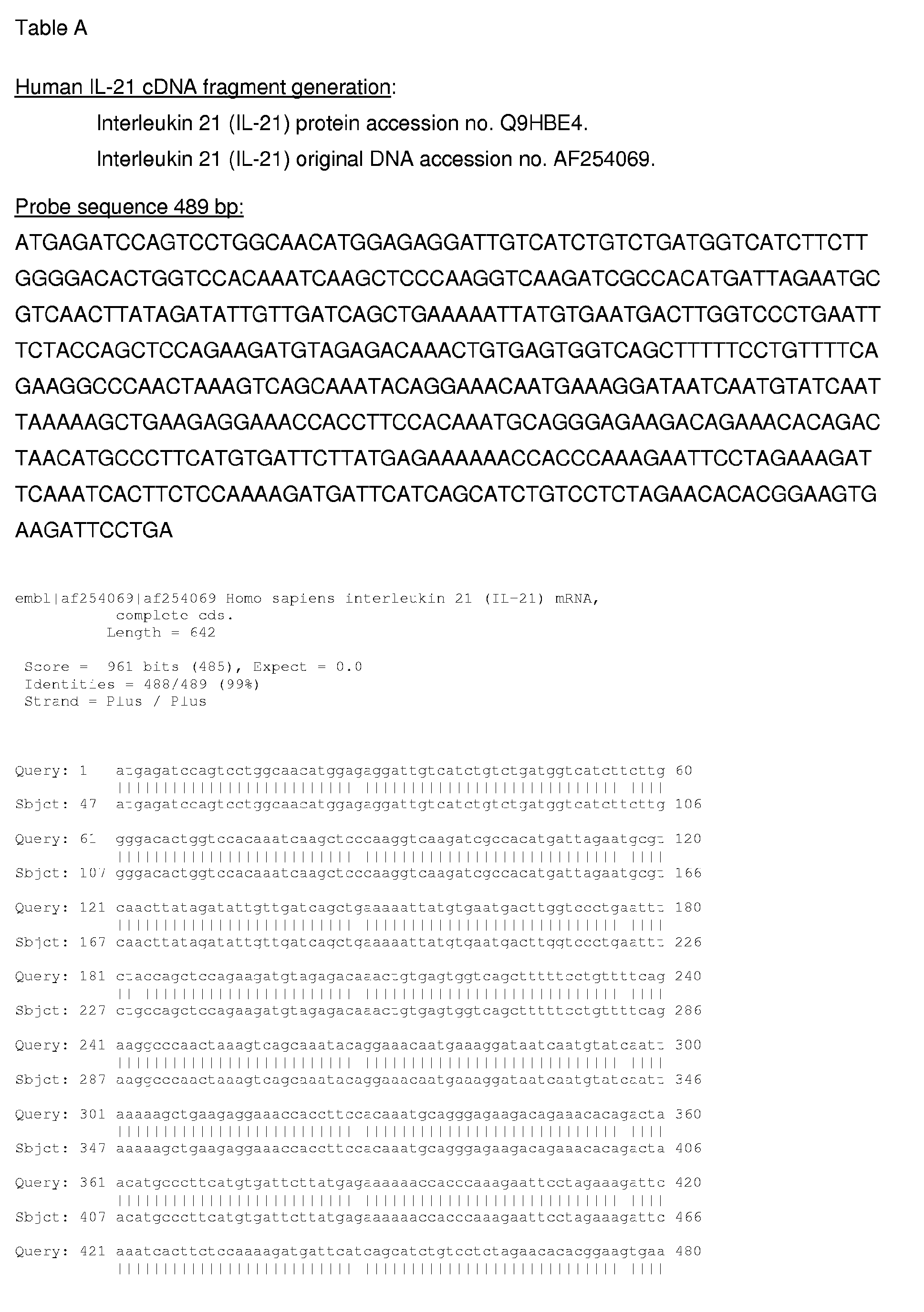

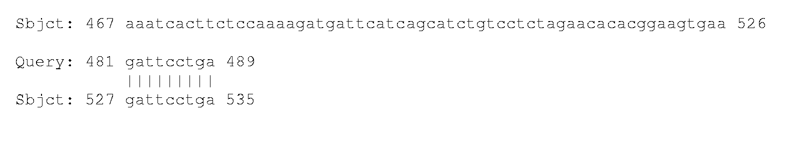

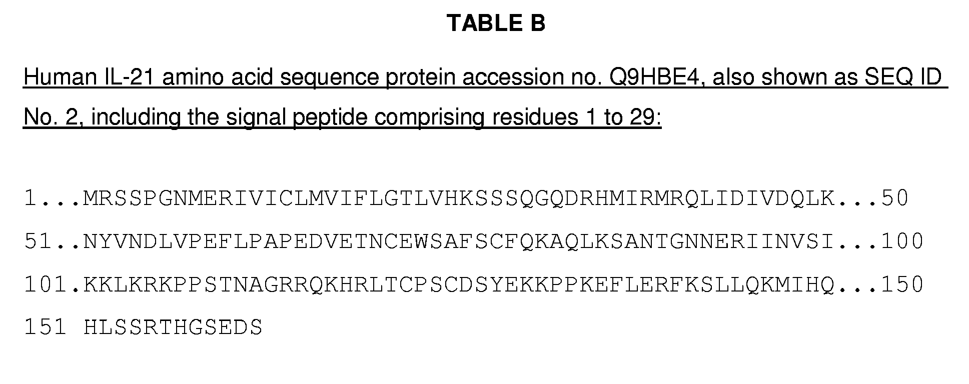

## Claims

1. Use of IL-21 for the manufacture of a medicament for the treatment of diseases or conditions where eosinophils are involved in a protective response in a subject in need thereof.

2. Use of IL-21 for the manufacture of a medicament for the treatment of allergic conditions in a subject in need thereof.

3. Use according to claim 1 and claim 2 wherein the allergic condition are asthma, allergic rhinitis or allergic diseases in the skin.

4. Use of IL-21 for the manufacture of a medicament for the treatment of parasitic diseases in a subject in need thereof.

5. Use according to claim 4, where the parasitic disease is a helminthic infection.

6. Use of an IL-21 polypeptide having a sequence identity to the polypeptide of SEQ ID No: 2, or to residues 30 to 162 of SEQ ID No: 2, of at least 70% for the manufacture of a medicament for the treatment of diseases or conditions where eosinophils are involved in a protective response in a subject in need thereof.

7. Use of an IL-21 polypeptide having a sequence identity to the polypeptide of SEQ ID No: 2, or to residues 30 to 162 of SEQ ID No: 2, of at least 70% for the manufacture of a medicament for the treatment of allergic conditions in a subject in need thereof.

8. Use according to claim 7 wherein the allergic conditions are asthma, allergic rhinitis or allergic diseases in the skin.

9. Use of an IL-21 polypeptide having a sequence identity to the polypeptide of SEQ ID No: 2, or to residues 30 to 162 of SEQ ID No: 2, of at least 70% for the manufacture of a medicament for the treatment of parasitic diseases in a subject in need thereof.

10. Use according to claim 9, where the parasitic disease is a helminthic infection.

11. Use according to any of claims 6 to 10, wherein the IL-21 polypeptide has a sequence identity to the polypeptide of SEQ ID No: 2, or to residues 30 to 162 of SEQ ID No: 2, of at least 80%.

12. Use according to claim 11, wherein the IL-21 polypeptide has a sequence identity to the polypeptide of SEQ ID No: 2, or to residues 30 to 162 of SEQ ID No: 2, of at least 90%.

13. Use according to claim 12, wherein the IL-21 polypeptide has a sequence identity to the polypeptide of SEQ ID No: 2, or to residues 30 to 162 of SEQ ID No: 2, of at least 95%.


### Annex to the application documents - subsequently filed sequences listing

SEQUENCE LISTING

<110>  Novo Nordisk A/S

<120>  TREATMENT OF ALLERGIC CONDITIONS BY USE OF IL 21

<130>  6581.215-EP

<160>  2

<170>  PatentIn version 3.3

<210>  1
<211>  488
<212>  DNA
<213>  Homo sapiens

<400>  1
atgagatcca gtcctggcaa catggagagg attgtcatct gtctgatggt catcttctgg      60

ggacactggt ccacaaatca agctcccaag gtcaagatcg ccacatgatt agaatgcgtc     120

aacttataga tattgttgat cagctgaaaa attatgtgaa tgacttggtc cctgaatttc     180

tgccagctcc agaagatgta gagacaaact gtgagtggtc agctttttcc tgttttcaga     240

aggcccaact aaagtcagca aatacaggaa acaatgaaag gataatcaat gtatcaatta     300

aaaagctgaa gaggaaacca ccttccacaa atgcagggag aagacagaaa cacagactaa     360

catgcccttc atgtgattct tatgagaaaa aaccacccaa agaattccta gaaagattca     420

aatcacttct ccaaaagatg attcatcagc atctgtcctc tagaacacac ggaagtgaag     480

attcctga                                                              488

<210>  2
<211>  162
<212>  PRT
<213>  Homo sapiens

<400>  2

Met Arg Ser Ser Pro Gly Asn Met Glu Arg Ile Val Ile Cys Leu Met
1               5                   10                  15

Val Ile Phe Leu Gly Thr Leu Val His Lys Ser Ser Ser Gln Gly Gln
            20                  25                  30

Asp Arg His Met Ile Arg Met Arg Gln Leu Ile Asp Ile Val Asp Gln
        35                  40                  45

Leu Lys Asn Tyr Val Asn Asp Leu Val Pro Glu Phe Leu Pro Ala Pro
    50                  55                  60

Glu Asp Val Glu Thr Asn Cys Glu Trp Ser Ala Phe Ser Cys Phe Gln
65                  70                  75                  80

Lys Ala Gln Leu Lys Ser Ala Asn Thr Gly Asn Asn Glu Arg Ile Ile
                85                  90                  95

Asn Val Ser Ile Lys Lys Leu Lys Arg Lys Pro Pro Ser Thr Asn Ala
            100                 105                 110

Gly Arg Arg Gln Lys His Arg Leu Thr Cys Pro Ser Cys Asp Ser Tyr
        115                 120                 125

Glu Lys Lys Pro Pro Lys Glu Phe Leu Glu Arg Phe Lys Ser Leu Leu
    130                 135                 140

Gln Lys Met Ile His Gln His Leu Ser Ser Arg Thr His Gly Ser Glu
145                 150                 155                 160

Asp Ser